# EUROPÄISCHE PATENTSCHRIFT

(11) **EP 0 781 761 B1**
(45) Veröffentlichungstag und Bekanntmachung des Hinweises auf die Patenterteilung: **21.03.2001**
(21) Anmeldenummer: 96117239.2
(22) Anmeldetag: 28.10.1996
(51) Int. Cl.: C07C 209/84, C07C 211/55, B01D 11/02

(54) **Verfahren und Vorrichtung zur Reinigung von handelsüblichem Diphenyl-p-phenylendiamin (DPPD)**
Process and aparatus for the purification of technical diphenyl-p-phenylenediamine
Procédé et dispositif de purification de diphényle-p-phénylènediamine de qualité technique

(30) Priorität: 28.12.1995 DE 19549032
(43) Veröffentlichungstag der Anmeldung: 02.07.1997
(73) Patentinhaber: Degussa-Hüls Aktiengesellschaft, 60287 Frankfurt am Main (DE)
(72) Erfinder: Frings, Albert-Johannes, Dr., 79618 Rheinfelden (DE); Horn, Michael, Dr., 79618 Rheinfelden (DE); Jenkner, Peter, Dr., 79618 Rheinfelden (DE); Monkiewicz, Jaroslaw, Dr., 79618 Rheinfelden (DE); Srebny, Hans-Günther, Dr., 48249 Dülmen (DE); Standke, Burkhard, Dr., 79540 Lörrach (DE); Trautvetter, Bertram, 79618 Rheinfelden (DE)

(56) Entgegenhaltungen:
- CHEMICAL ABSTRACTS, vol. 89, no. 3, 17.Juli 1978 Columbus, Ohio, US; abstract no. 23925, BURMISTROV, S. I. ET AL: "Purification of N,N'-diphenyl-p-phenylenediamine" XP002029089 & SU 595 295 A (DNEPROPETROVSK CHEMICAL-TECHNOLOGICAL INSTITUTE, USSR)

## Beschreibung

Die vorliegende Erfindung betrifft ein Verfahren und eine Vorrichtung zur Reinigung von rohem Diphenyl-p-phenylendiamin (DPPD) und die Verwendung von gereinigtem DPPD.

DPPD wird u. a. bei der Reaktion von Kaliummethacrylat mit 3-Chlorpropyltrimethoxysilan großtechnisch als Stabilisator verwendet. Der Einsatz von handelsüblichem DPPD kann hier die Produktqualität beeinträchtigen, insbesondere im Hinblick auf die Farbzahl. Leider sind industrielle Mengen an DPPD am Markt bisher nur in technischer Qualität verfügbar.

Das rohe DPPD - d. h. handelsüblich bzw. technisch rein - besitzt in der Regel eine Reinheit im Bereich um 85 Gew.-%, der Rest von rd. 15 Gew.-% besteht im wesentlichen aus nicht oder unvollständig umgesetzten Edukten, wie z. B. Diphenylamin, und aus anorganischen Komponenten, wie Eisen sowie Chlor, beispielsweise in Form von Eisenchlorid. Die Handelsware ist ein graues Material, das in Form eines Pulvers oder in Form von Schuppen angeboten wird. Im Unterschied dazu ist reines DPPD weiß und kristallin.

In der Literatur werden mehrere Methoden zur Reinigung von DPPD beschrieben. So ist es möglich, DPPD aus Chlorkohlenwasserstoffen oder Tetrachlorkohlenstoff zu kristallisieren (Beilstein 13, IV, 116). Nachteilig bei diesen Verfahren ist allerdings der Einsatz von giftigen und umweltschädlichen chlorierten Lösemitteln.

Feiner geht aus Chemical Abstracts-CA:23925g (1989), vgl, SU-A5952995, ein mehrsfufiges Verfahren zur Reinigung von DPPD hervor.

Eine weitere Reinigungsmethode ist die Feststoffdestillation. Diese Methode kann im Labor erfolgreich angewendet werden. Für die Reinigung von rohem DPPD im industriellen Maßstab ist die Feststoffdestillation allerdings kein geeignetes Verfahren, da technisch zu aufwendig.

Der vorliegenden Erfindung lag daher die Aufgabe zugrunde, ein Verfahren bereitzustellen, das es ermöglicht, rohes DPPD in einfacher und wirtschaftlicher Weise zu reinigen und für Anwendungen im industriellen Maßstab bereitzustellen.

Überraschenderweise wurde gefunden, daß man durch Extrahieren von Roh-DPPD mit einem Kohlenwasserstoff oder einem Gemisch aus Kohlenwasserstoffen, Überleiten des Extraktes über eine Adsorptionsschicht und daß man reines DPPD, vorzugsweise weiß und in kristalliner Form, in einfacher und wirtschaftlicher Weise aus der dabei anfallenden Lösung gewinnt. Im vorliegenden Verfahren kann man als Roh-DPPD auch technisches DPPD einsetzen.

Gegenstand der vorliegenden Erfindung ist daher ein Verfahren zur Reinigung von rohem N,N'-Diphenyl-p-phenylendiamin (DPPD), das dadurch gekennzeichnet ist, daß man Roh-DPPD mit einem Kohlenwasserstoff oder einem Kohlenwasserstoffgemisch extrahiert, das Extrakt über eine Adsorptionsschicht leitet und gereinigtes DPPD aus dieser Lösung gewinnt. Vorzugsweise führt man die Reinigung unter Schutzgas durch.

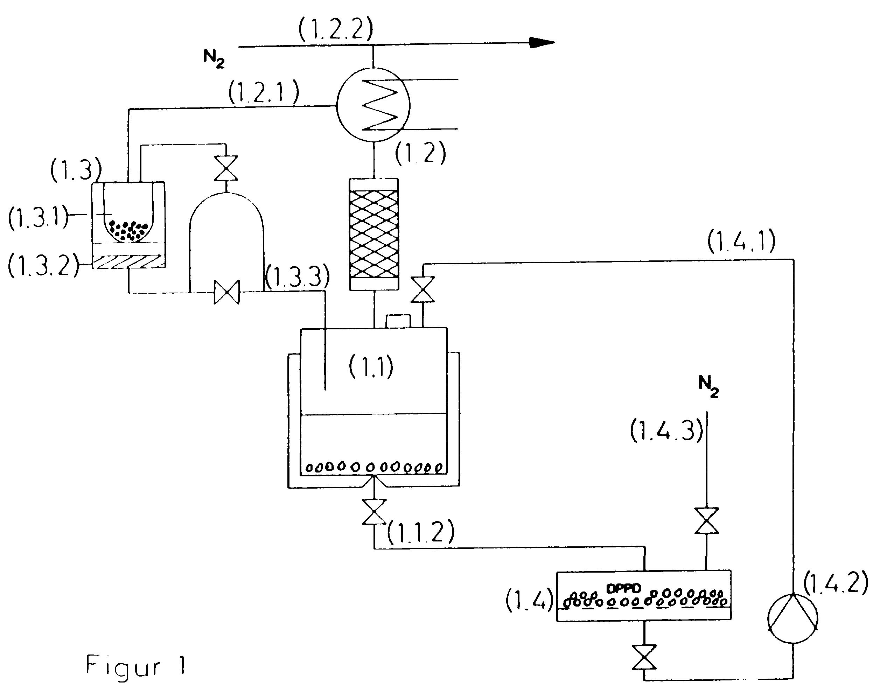

In Figur 1 ist das Fließschema einer bevorzugten Ausführungsform der Vorrichtung gezeigt, in der das erfindungsgemäße Verfahren durchgeführt werden kann.

Gegenstand ist daher auch eine Vorrichtung zur Reinigung von N,N'-Diphenyl-p-phenylendiamin (DPPD), welche gekennzeichnet ist durch einen Lösemittelverdampfer (1.1), einen nachgeschalteten Kondensator (1.2), der über eine Lösemittelleitung (1.2.1) mit einer Extraktionseinheit (1.3) verbunden ist, die ihrerseits mit einem innenliegenden und Flüssigkeiten durchlassenden Behältnis für die Aufnahme von Roh-Produkt (1.3.1) und einer nachfolgenden Adsorptionseinheit (1.3.2) ausgestattet ist, die über eine Extraktleitung (1.3.3) mit dem Lösemittelverdampfer (1.1) verbunden ist, der seinerseits bodenseitig über eine Schleuse (1.1.2) mit einer Filtereinheit (1.4) gekoppelt ist, wobei eine Filtratleitung (1.4.1) über eine Pumpe (1.4.2) zu dem Lösemittelverdampfer (1.1) führt. Geeigneterweise wird für die erfindungsgemäße Vorrichtung auch eine oder mehrere Schutzgaszuleitung(en) (1.2.2 und/oder 1.4.3) vorgesehen.

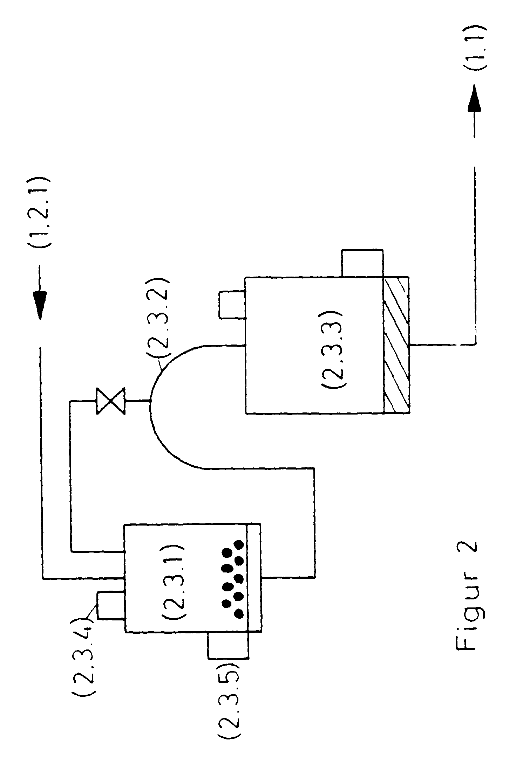

Bei der in Figur 2 gezeigten Ausführungsform einer bevorzugten, erfindungsgemäßen Vorrichtung ist vorgesehen, daß die Extraktionseinheit aus einem Flüssigkeiten durchlassenden Extraktionsbehälter (2.3.1) für die Aufnahme von Roh-Produkt und einem hiermit über ein Leitungssystem (2.3.2) verbundenen Behälter (2.3.3), der ein Adsorptionsmittel enthält, besteht. Geeigneterweise ist die Extraktionseinheit mit einer Roh-Produktaufgabe (2.3.4) und/oder einem Rückstandsauslaß (2.3.5) ausgestattet. Darüber hinaus kann aber auch der Lösemittelverdampfer (1.1), die Extraktionseinheit (1.3) sowie die Filtrationseinheit (1.4) mit einer Materialaufgabe- bzw. einer Material-entnahmeschleuse ausgestattet sein.

Um beispielsweise eine bessere Durchmischung in den vorliegenden Fest-/Flüssigphasen oder auch einen besseren Wärmeübergang in den Flüssigphasen zu erzielen, kann man eine erfindungsgemäße Vorrichtung bezüglich der Einheiten (1.3.1), (2.3.1) sowie (1.1) auch mit einer Rührer- oder Mischereinheit ausstatten.

Hinsichtlich der Gewinnung von gereinigtem DPPD kann eine erfindungsgemäße Vorrichtung anstelle der Filtrationseinheit (1.4) auch mit anderen, an sich bekannten Trennverfähren für die Trennung von Fest/Flüssig-Systemen ausgestattet sein, z. B. mit einer Zentrifuge oder einem Dekanter.

Im allgemeinen führt man das erfindungsgemäße Verfahren wie folgt durch:

Im Lösemittelverdampfer (1.1) können Kohlenwasserstoffe oder Kohlenwasserstoffgemische vorgelegt werden. Die Menge an Extraktionsmittel, die hier vorgelegt wird, richtet sich in der Regel nach der Größe der zur Verfügung stehenden Apparatur oder Anlage.

Im allgemeinen sind alle cyclischen, aliphatischen oder aromatischen, aber auch acyclischen, verzweigte oder unverzweigte Kohlenwasserstoffe mit 3 bis 10 Kohlenstoffatomen geeignet. Zur Durchführung der Extraktion werden vorzugsweise Pentan, Cyclopentan, Hexan, Cyclohexan, Benzol, Toluol, Heptan, Cycloheptan u. v. m. eingesetzt. Im erfindungsgemäßen Verfahren extrahiert man besonders vorzugsweise mit Hexan, wobei hier das Hexan für alle Isomeren des Hexans sowie seiner Isomerengemische steht. Aber auch ungesättigte cyclische bzw. acyclische Kohlenwasserstoffe, wie Cyclopenten, Penten, Cyclohexen, Cyclohexadien, Hexen, u. v. m. können beim erfindungsgemäßen Verfahren als Extraktionsmittel dienen. Ebenso können auch Gemische von aliphatisch gesättigten, aliphatisch ungesättigten, cyclischen, acyclischen und aromatischen Kohlenwasserstoffen für das erfindungsgemäße Verfahren eingesetzt werden.

In der Regel wird der Lösemittelverdampfer (1.1) bei einer Temperatur im Bereich von 40 bis 110°C unter Normaldruck betrieben. Der so in die Gasphase überführte Lösemittelanteil wird geeigneterweise über einen Kondensator (1.2) gefahren und das dabei anfallende Kondensat über eine Lösemittelleitung dem Extraktionsbehälter (1.3) zugeführt.

Der gegenüber Flüssigkeiten durchlässige Extraktionsbehälter (1.3.1 sowie 2.3.1) sowie der Boden zur Aufnahme eines Adsorptionsmittels (1.3.2 sowie 2.3.3) der Extraktionseinheit können aus einem textilen Gewebe, das aus einem Polyester, Polyamid oder einem anderen Polymer gefertigt sein kann, und/oder aus einem Metallgewebe bestehen. Die Maschenweite solcher Gewebe liegt üblicherweise im Bereich von 0,01 bis 0,2 mm. Der gegenüber Flüssigkeiten durchlässige Extraktionsbehälter bzw. Boden zur Aufnahme eines Adsorptionsmittels kann aber auch aus keramischen Werkstoffen bestehen, z. B. aus einer G 1 - G 4 Fritte.

Der Teil der Extraktionseinheit (1.3.2 sowie 2.3.3), der geeigneterweise mit einem Flüssigkeiten durchlassenden Boden ausgerüstet ist, kann mit einem Adsorptionsmittel überschichtet werden. Beim erfindungsgemäßen Verfahren setzt man als Adsorptionsmittel vorzugsweise Kieselgel ein, beispielsweise ein Kieselgel der Fa. MERCK. Für solche Adsorptionsschichten werden Schichtdicken im Bereich von 2 cm bis 30 cm bevorzugt, die Schichten können aber auch stärker sein. So kann man beispielsweise Kieselgel mit einer mittleren Korngröße von 0,063 bis 3,00 mm hier einsetzen, bevorzugt wird Kieselgel mit einer mittleren Korngröße von 0,063 bis 0,5 mm eingesetzt. Besonders vorzugsweise setzt man beim erfindungsgemäßen Verfahren ein Kieselgel mit einer mittleren Korngröße von 0,20 mm bis 0,5 mm ein, insbesondere solches mit einer BET-Oberfläche von 450 bis 550 m²/g. Ein Flüssigkeiten durchlassender Extraktionsbehälter (1.3.1), der mit handelsüblichem DPPD gefüllt ist, kann über der Adsorptionsschicht (1.3.2) angeordnet werden. Die auf diese Weise präparierte Extraktionseinheit (1.3) wird im allgemeinen gasdicht verschlossen und über die Kondensatleitung (1.2.1) mit Lösemittel beaufschlagt.

Der Extraktionsbehälter (1.3.1) und der Behälter für das Adsorptionsmaterial (1.3.2) können selbstverständlich auch voneinander getrennt angeordnet werden, wobei das Roh-Extrakt aus dem Extraktionsbehälter (2.3.1) über ein Leitungssystem (2.3.2) dem Behälter für das Adsorptionsmaterial (2.3.3) zugeführt werden kann; der Figur 2 ist hierzu eine bevorzugte Ausführungsform zu entnehmen.

Geeigneterweise ist das im Extraktionsbehälter (1.3.1) oder auch das in einer Vorrichtungseinheit (2.3.1) vorgelegte Roh-DPPD mit Extraktionsmittel bedeckt. Der Extraktionsbehälter (1.3 sowie 2.3.1) füllt sich aber in der Regel bis zu einer Höhe, die der höchsten Stelle in der Extraktabführung (1.3.3 bzw. 2.3.2) entspricht. Wird dieses Niveau erreicht, fließt das Extrakt durch die Heberwirkung in den Lösemittelverdampfer (1.1) bzw. über die Adsorptionseinheit (2.3.3) in den Lösmittelverdampfer (1.1) ab.

Beim erfindungsgemäßen Verfahren extrahiert man im allgemeinen bei einer Temperatur im Bereich von 40 bis 100°C. Auch die Adsorption wird in der Regel bei einer Temperatur im Bereich von 40 bis 100°C durchgeführt.
Die Adsorptionsschicht (1.3.2 sowie 2.3.3) nimmt dabei in geeigneter Weise im Extrakt enthaltene Partikel und Trübungen verursachende Verunreinigungen auf, so daß die in den Lösemittelverdampfer (1.1) abfließende Lösung klar ist. Das erfindungsgemäße Verfahren kann sowohl unter Normaldruck, bei vermindertem Druck oder auch bei leicht erhöhtem Druck durchgeführt werden.

Es hat sich gezeigt, daß es zweckmäßig sein kann, die Extraktionseinheit (1.3 sowie 2.3.1 und 2.3.3) einschließlich der Leitungen (2.3.2 sowie 1.3.3 und 1.1.2) gegen Wärmeverluste zu isolieren. Für die Extraktionseinheit (1.3 sowie 2.3.1 und 2.3.3) einschließlich der Leitungen (2.3.2 sowie 1.3.3 und 1.1.2) kann auch eine Begleitbeheizung vorgesehen werden.

Die Aufgabe von Roh-DPPD in den Extraktionsbehälter (1.3.1 sowie 2.3.1) kann diskontinuierlich oder kontinuierlich erfolgen. Insbesondere zur kontinuierlichen Durchführung des erfindungsgemäßen Verfahrens kann der Extraktionsbehälter (1.3 sowie 2.3.1) mit einer Produktaufgabevorrichtung für Roh-DPPD (z. B.: 2.3.4) sowie einer Austragsvorrichtung für den Extraktionsrückstand (z. B.: 2.3.5) ausgerüstet werden. Auch die Adsorptionsmittelbehälter (z. B.: 2.3.3) können für die Entnahme von verbrauchtem Kieselgel und die Aufgabe von frischem Kieselgel mit geeigneten Schleusen ausgerüstet werden. Vorteilhaft ist eine derartige Anordnung zur Steigerung der Anlagenkapazität für die Herstellung von gereinigtem DPPD.

Die erfindungsgemäße Vorrichtung wird vorteilhafterweise mit Inertgas, z. B. Stickstoff, überschleiert (1.2.2 bzw. 1.4.3), so daß keine zündfähigen Gemische in der Anlage auftreten können.

Nach mehreren Extraktionscyclen kann man beobachten, wie im Lösemittelverdampfer (1.1) gereinigtes DPPD als weißes, mikrokristallines Pulver aus der übersättigten Lösung auskristallisiert.

Das erfindungsgemäße Verfahren zur Reinigung von Roh-DPPD kann diskontinuierlich sowie kontinuierlich durchgeführt werden. Der Extraktionsbehälter (1.3.1 sowie 2.3.1) wird bei diskontinuierlichem Betrieb der Anlage geeigneterweise mehrfach entleert und wieder mit Roh-DPPD gefüllt, bevor man die Extraktion abbricht und das gereinigte Produkt aus dem Extraktionsmittel isoliert. Bevorzugt sind hierbei 2 oder 3 Entleerungs- bzw. Füllvorgänge, erst dann wird das reine DPPD isoliert. Die Adsorptionsschicht (1.3.2 sowie 2.3.3) kann teilweise oder ganz ausgetauscht werden Es ist jedoch auch möglich, mehrere Extraktionen über die gleiche Adsorptionsschicht zu fahren, ohne daß Verunreinigungen in den Lösemittelverdampfer (1.1) gelangen. Die Extraktionsdauer beträgt in der Regel 20 bis 60 Stunden, so daß im allgemeinen auch die mittlere Verweilzeit des Produktes im Reinigungsprozeß 20 bis 60 Stunden beträgt. Die Erfahrung zeigt, daß eine Extraktionsdauer von ca. 35 Stunden bezüglich Raum-Zeit-Ausbeuten zu bevorzugen ist. Beim erfindungsgemäßen Verfahren erreicht man im allgemeinen Ausbeuten von 60 bis 85 %, bezogen auf das eingesetzte Roh-DPPD, d. h., daß das erfindungsgemäße Verfahren auch ohne Wertproduktverlust betrieben werden kann.

Nach Beendigung der Extraktion wird die Suspension aus Extraktionsmittel und gereinigtem, kristallinem DPPD beispielsweise auf einen Filter (1.4) abgelassen und getrennt. Das Filtrat ist klar und kann im allgemeinen auch ohne weitere Reinigung für weitere Extraktionen eingesetzt werden (vgl. 1 4.1 und 1.4.2). Das Filtrat kann aber auch vor einer Wiederverwertung erst über eine Destillation gefahren werden.

Das gereinigte, vorliegende DPPD kann beispielsweise im Vakuum getrocknet oder unter Einleitung von Stickstoff als weißes bis hellbeiges mikrokristallines Pulver isoliert werden. Das nach dem erfindungsgemäßen Verfahren gereinigte Produkt weist im allgemeinen eine Reinheit von > 90 %, vorzugsweise > 95 % und ganz besonders vorzugsweise 97 bis 99 % (Bestimmung durch NMR-Spektroskopie) auf. Das erfindungsgemäß gereinigte DPPD weist vorzugsweise einen Schmelzbereich im Temperaturbereich von 142 °C bis 154 °C auf, besonders bevorzugt ist gereinigtes DPPD mit einem Schmelzbereich im Temperaturbereich von 144 °C bis 148 °C. Im Unterschied dazu hat das handelsübliche Roh-DPPD mit einer Reinheit von rd. 85 % einen Schmelzbereich von 124 bis 126 °C.

Setzt man DPPD, das gemäß dem vorliegenden Verfahren gereinigt wurde, beispielsweise für die Stabilisierung von MEMO (3-Methacryloxypropyltrimethoxysilan) ein (vgl. DE-PS 38 32 621), zeichnet sich das so erhaltene Produkt durch eine besonders hervorragende Farbzahl aus.

Ein weiterer Vorteil des erfindungsgemäßen Verfahrens ist, daß durch den Einsatz weitgehend umweltverträglicher Kohlenwasserstoffe auf Methoden, die Chlorkohlenwasserstoffe als Extraktionsmittel einsetzen, verzichtet werden kann.

Die vorliegende Erfindung wird durch die nachfolgenden Beispiele näher erläutert:

### Beispiele

### Beispiel 1

Der Extraktionsbehälter, ausgerüstet mit Flüssigkeiten durchlassendem Boden, wird mit 5,5 kg Kieselgel 60 (Korngröße 0,2 bis 0,5 mm) der Fa. MERCK, das mit 4 kg Keramiksattelkörpern mit einem Durchmesser von 15 mm vermischt ist, befüllt. Über dieser Adsorptionsschicht wird ein Flüssigkeiten durchlassender Extraktionsbehälter, der 10 kg rohes DPPD der Fa. GÖBEL und PFRENGLE enthält, am Deckel des Extraktionsbehälters aufgehängt.

In den Lösemittelverdampfer werden 300 l Hexan (Isomerengemisch mit einem Siedepunkt von 68 °C) der Fa. ÖLFABRIK Lahr vorgelegt Die Anlage wird permanent mit trockenem Stickstoff überschleiert, so daß Luftsauerstoff und Luftfeuchtigkeit ausgeschlossen werden können.

Das Extraktionsmittel wird zum Sieden erhitzt. Man mißt in der Blase eine Temperatur von 68 °C. Das Kondensat fließt über die Kondensatleitung in den Extraktionsbehälter und von dort in Abhängigkeit vom Füllstand im Extraktionsbehälter mit gelöstem DPPD in den Lösemittelverdampfer zurück. Die Extraktionscyclen werden über einen Zeitraum von 60 h wiederholt. Danach öffnet man die Extraktionseinheit zur Entnahme des Extraktionsbehälters. Man entfernt den Rückstand aus dem Extraktionsbehälter Die Auswaage ergibt nach Trocknung eine Rückstandsmenge von 1,5 kg. Das Kieselgel wird gegen 6 kg neues Kieselgel, 4 kg Sattelkörper enthaltend, ausgetauscht. Der Extraktionsbehälter wird, mit 10 kg DPPD roh gefüllt, wieder in den Extraktionsbehälter eingesetzt. Nach einer Extraktionsdauer von 36 h erhält man 1,5 kg Rückstand.

Der Inhalt aus dem Lösemittelverdampfer wird auf einen Filter abgelassen. Der Filterkuchen wird 2mal mit ca. 18 kg Hexan gewaschen und im Vakuum bei gleichzeitigem Einleiten von wenig Stickstoff getrocknet. Man erhält 16 kg DPPD rein als hellbeiges mikrokristallines Pulver. Dies entspricht einer Ausbeute von 80 %, bezogen auf die gesamte Einsatzmenge an rohem DPPD

### Beispiel 2

Einsatzmengen für eine Extraktion:
4 kg Kieselgel 60 (0,2 bis 0,5 mm) = 55 mm Schichthöhe
1,5 kg gebrauchtes Kieselgel (gelb) = 25 mm Schichthöhe
4 kg Sattelkörper: Gesamthöhe der Adsorptionsschicht = 80 mm
400 l Hexan (Isomerengemisch)
9 kg DPPD roh (Schuppen)

Nach 40,5 h Extraktionsdauer ergab die Rückstandsbestimmung 2,5 kg. 6,5 kg waren extrahiert worden.

### Beispiel 3

Einsatzmenge für die erste Extraktion:
4 kg Kieselgel
1,5 kg gebrauchtes Kieselgel
9 kg DPPD roh (Pulver)
400 l Hexan

Einsatzmengen für die zweite Extraktion:
3 kg ungebrauchtes Kieselgel 60
3 kg gebrauchtes Kieselgel 60
4 kg Sattelkörper
10 kg DPPD roh (Schuppen)

Isoliert wurden aus 19 kg DPPD roh 12,5 kg = 67 % DPPD rein in Form eines weißen Pulvers.

### Legende zu Figur 1:

Fließschema einer bevorzugten Ausführungsform der Vorrichtung für das Verfahren zur Reinigung von Roh-DPPD.

### Legende zu Figur 2:

Eine weitere bevorzugte Vorrichtung zur Reinigung von Roh-DPPD insbesondere hinsichtlich der Ausführungsform der Extraktionseinheit, vgl. hierzu auch Figur 1.

## Patentansprüche

1. Verfahren zur Reinigung von rohem N,N'-Diphenyl-p-phenylendiamin (DPPD),
dadurch gekennzeichnet,
daß man Roh-DPPD mit einem Kohlenwasserstoff oder einem Kohlenwasserstoffgemisch extrahiert, das Extrakt über eine Adsorptionsschicht leitet und gereinigtes DPPD aus dieser Lösung gewinnt.

2. Verfahren nach Anspruch 1,
dadurch gekennzeichnet,
daß man die Reinigung unter Schutzgas durchführt.

3. Verfahren nach Anspruch 1 oder 2,
dadurch gekennzeichnet,
daß man technisches DPPD einsetzt.

4. Verfahren nach mindestens einem der Ansprüche 1 bis 3,
dadurch gekennzeichnet,
daß man bei einer Temperatur im Bereich von 40 bis 100°C extrahiert.

5. Verfahren nach mindestens einem der Ansprüche 1 bis 4,
dadurch gekennzeichnet,
daß man mit Hexan extrahiert.

6. Verfahren nach mindestens einem der Ansprüche 1 bis 5,
dadurch gekennzeichnet,
daß man als Adsorptionsmittel Kieselgel einsetzt.

7. Verfahren nach Anspruch 6,
dadurch gekennzeichnet,
daß man ein Kieselgel mit einer BET-Oberfläche von 450 bis 550 m²/g einsetzt.

8. Verfahren nach Anspruch 6 oder 7,
dadurch gekennzeichnet,
daß man ein Kieselgel mit einer mittleren Korngröße von 0,063 bis 0,5 mm einsetzt.

9. Verfahren nach mindestens einem der Ansprüche 1 bis 8,
dadurch gekennzeichnet,
daß man die Adsorption bei einer Temperatur im Bereich von 40 bis 100°C durchführt.

10. Verfahren nach mindestens einem der Ansprüche 1 bis 9,
dadurch gekennzeichnet,
daß die mittlere Verweilzeit des Produktes im Reinigungsprozeß 20 bis 60 Stunden beträgt.

11. Verfahren nach mindestens einem der Ansprüche 1 bis 10,
dadurch gekennzeichnet,
daß die Reinigung von Roh-DPPD kontinuierlich durchgeführt wird.

12. Verfahren nach mindestens einem der Ansprüche 1 bis 11,
dadurch gekennzeichnet,
daß das gereinigte DPPD eine Reinheit von > 90 % aufweist.

13. Verfahren nach Anspruch 12,
dadurch gekennzeichnet,
daß das gereinigte DPPD eine Reinheit von > 95 % aufweist.

14. Verfahren nach mindestens einem der Ansprüche 1 bis 13,
dadurch gekennzeichnet,
daß das gereinigte DPPD einen Schmelzbereich im Temperaturbereich von 142 bis 148 °C aufweist.

15. Vorrichtung zur Reinigung von N,N'-Diphenyl-p-phenylendiamin (DPPD)
nach Anspruch 1, gekennzeichnet durch
einen Lösemittelverdampfer (1.1), einen nachgeschalteten Kondensator (1.2), der über eine Lösemittelleitung (1.2.1) mit einer Extraktionseinheit (1.3) verbunden ist, die ihrerseits mit einem innenliegenden und Flüssigkeiten durchlassenden Behältnis für die Aufnahme von Roh-Produkt (1.3.1) und einer nachfolgenden Adsorptionseinheit (1.3.2) ausgestattet ist, die über eine Extraktleitung (1.3.3)
mit dem Lösemittelverdampfer (1.1) verbunden ist, der seinerseits bodenseitig über eine Schleuse (1.1.2) mit einer Filtereinheit (1.4) gekoppelt ist, wobei eine Filtratleitung (1.4.1) über eine Pumpe (1.4.2) zu dem Lösemittelverdampfer (1.1) führt.

16. Vorrichtung nach Anspruch 15,
gekennzeichnet
durch eine oder mehrere Schutzgaszuleitungen (1.2.2 und/oder 1.4.3).

17. Vorrichtung nach Anspruch 15 oder 16,
dadurch gekennzeichnet,
daß die Extraktionseinheit aus einem Flüssigkeiten durchlassenden Extraktionsbehälter (2.3.1) für die Aufnahme von Roh-Produkt und einem hiermit über ein Leitungssystem (2.3.2) verbundenen Behälter (2.3.3), der ein Adsorptionsmittel enthält, besteht.

18. Vorrichtung nach mindestens einem der Ansprüche 15 bis 17,
dadurch gekennzeichnet,
daß die Extraktionseinheit mit einer Roh-Produktaufgabe (2.3.4) und/oder einem Rückstandsauslaß (2.3.5) ausgestattet ist.

## Claims

1. A process for purifying crude N,N'-diphenyl-p-phenylenediamine (DPPD), characterized in that crude DPPD is extracted with a hydrocarbon or hydrocarbon mixture, the extract is passed over an adsorption layer and purified DPPD is recovered from this solution.

2. A process according to claim 1, characterized in that the purification is carried out under a blanketing gas.

3. A process according to claim 1 or 2, characterized in that technical-grade DPPD is used.

4. A process according to at least one of claims 1 to 3, characterized in that the extraction is conducted at a temperature in the range from 40 to 100°C.

5. A process according to at least one of claims 1 to 4, characterized in that the extractant is hexane.

6. A process according to at least one of claims 1 to 5, characterized in that the adsorbent is silica gel.

7. A process according to claim 6, characterized in that the silica gel used has a BET surface area of from 450 to 550 m²/g.

8. A process according to claim 6 or 7, characterized in that the silica gel used has an average particle size of from 0.063 to 0.5 mm.

9. A process according to at least one of claims 1 to 8, characterized in that the adsorption is carried out at a temperature in the range from 40 to 100°C.

10. A process according to at least one of claims 1 to 9, characterized in that the average residence time of the product in the purification process is from 20 to 60 hours.

11. A process according to at least one of claims 1 to 10, characterized in that the purification of crude DPPD is carried out continuously.

12. A process according to at least one of claims 1 to 11, characterized in that the purified DPPD has a purity of > 90%.

13. A process according to claim 12, characterized in that the purified DPPD has a purity of > 95%.

14. A process according to at least one of claims 1 to 13, characterized in that the purified DPPD has a melting range from 142 to 148°C.

15. Apparatus for purifying N,N'-diphenyl-p-phenylenediamine (DPPD) according to claim 1, characterized by a solvent evaporator (1.1) , a downstream condenser (1.2) which is connected via a solvent line (1.2.1) to an extraction unit (1.3) which in turn is equipped with an interior and liquid-permeable container for receiving crude product (1.3.1) and a downstream adsorption unit (1.3.2) which is connected via an extract line (1.3.3) to the solvent evaporator (1.1) which in turn is coupled at the bottom via a lock (1.1.2) to a filter unit (1.4), a filtrate line (1.4.1) leading via a pump (1.4.2) to the solvent evaporator (1.1).

16. Apparatus according to claim 15, characterized by one or more blanketing gas lines (1.2.2 and/or 1.4.3).

17. Apparatus according to claim 15 or 16, characterized in that the extraction unit consists of a liquid-permeable extraction vessel (2.3.1) for receiving crude product and a vessel (2.3.3) which is connected thereto via a line system (2.3.2) and contains an adsorbent.

18. Apparatus according to at least one of claims 15 to 17, characterized in that the extraction unit is equipped with a crude product feeder (2.3.4) and/or a residue outlet (2.3.5).

## Revendications

1. Procédé de purification de la N,N'-diphényl-p-phénylè-nediamine (DPPD) brute,
caractérisé en ce qu'
on extrait la DPPD brute avec un hydrocarbure ou un mélange d'hydrocarbures, on conduit l'extrait sur une couche d'adsorption et on obtient la DPPD purifiée à partir de cette solution.

2. Procédé selon la revendication 1,
caractérisé en ce qu'
on effectue la purification sous gaz protecteur.

3. Procédé selon la revendication 1 ou 2,
caractérisé en ce qu'
on met en oeuvre du DPPD technique.

4. Procédé selon au moins l'une quelconque des revendications 1 à 3,
caractérisé en ce qu'
on extrait à une température dans la zone de 40 à 100°C.

5. Procédé selon au moins l'une quelconque des revendications 1 à 4,
caractérisé en ce qu'
on extrait avec de l'hexane.

6. Procédé selon au moins l'une quelconque des revendications 1 à 5,
caractérisé en ce qu'
on met en oeuvre comme agent d'adsorption du gel de silice.

7. Procédé selon la revendication 6,
caractérisé en ce qu'
on met en oeuvre un gel de silice ayant une surface BET de 450 à 550 m²/g.

8. Procédé selon la revendication 6 ou 7,
caractérisé en ce qu'
on met en oeuvre un gel de silice ayant une taille de grains moyenne de 0,063 à 0,5 mm.

9. Procédé selon au moins l'une quelconque des revendications 1 à 8,
caractérisé en ce qu'
on effectue l'adsorption à une température dans la zone de 40 à 100°C.

10. Procédé selon au moins l'une quelconque des revendications 1 à 9,
caractérisé en ce que
le temps moyen de séjour du produit dans le processus de purification s'élève de 20 à 60 heures.

11. Procédé selon au moins l'une quelconque des revendications 1 à 10,
caractérisé en ce que
la purification de la DPPD brute s'effectue en continu.

12. Procédé selon au moins l'une quelconque des revendications 1 à 11,
caractérisé en ce que
la DPPD purifiée possède une pureté de > 90 %.

13. Procédé selon la revendication 12,
caractérisé en ce que
la DPPD purifiée possède une pureté de > 95 %.

14. Procédé selon au moins l'une quelconque des revendications 1 à 13,
caractérisé en ce que
la DPPD purifiée possède une zone de fusion dans la plage de températures de 142 à 148°C.

15. Dispositif de purification de la N,N'-diphényl-p-phénylènediamine (DPPD) selon la revendication 1, caractérisé par
un évaporateur pour solvants (1.1), un condenseur postconnecté (1.2) relié par une conduite pour solvants (1.2.1) à une unité d'extraction (1.3), pourvue de son côté d'un récipient situé à l'intérieur et qui fait passer les liquides pour la reprise du produit brut (1.3.1), et d'une unité d'adsorption (1.3.2) subséquente reliée par une conduite d'extraction (1.3.3) à l'évaporateur pour solvants (1.1), dont le fond est couplé par un sas (1.1.2) à une unité de filtre (1.4), une conduite pour le filtrat (1.4.1) étant reliée par l'intermédiaire d'une pompe (1.4.2) à l'évaporateur pour solvants (1.1).

16. Dispositif Belon la revendication 15,
caractérisé par
une ou plusieurs conduites d'amenée pour gaz protecteur (1.2.2 et/ou 1.4.3).

17. Dispositif selon la revendication 15 ou 16,
caractérisé en ce que
l'unité d'extraction consiste en un récipient d'extraction (2.3.1) qui laisse passer un liquide pour la reprise du produit brut et en un récipient (2.3.3) relié de cette façon par un système de conduites (2.3.2) qui contient un agent d'adsorption.

18. Dispositif selon au moins l'une quelconque des revendications 15 à 17,
caractérisé en ce que
l'unité d'extraction est équipée d'une alimentation en produit brut (2.3.4) et/ou d'une évacuation de résidu (2.3.5).
